(19) Europäisches Patentamt
European Patent Office
Office européen des brevets

(11) Numéro de publication: 0 297 641 B1

(12) # FASCICULE DE BREVET EUROPEEN

(45) Date de publication de fascicule du brevet: **22.01.92**

(51) Int. Cl.$^5$: **C07F 5/02**, C07C 279/02

(21) Numéro de dépôt: **88201152.1**

(22) Date de dépôt: **07.06.88**

(54) **Composés guanidiniques comprenant un ion tétraphénylborate substitué, procédé d'obtention de ces composés et utilisation des composés lors de la synthése peptidique.**

(30) Priorité: **19.06.87 FR 8708696**

(43) Date de publication de la demande: **04.01.89 Bulletin 89/01**

(45) Mention de la délivrance du brevet: **22.01.92 Bulletin 92/04**

(84) Etats contractants désignés: **AT BE CH DE ES FR GB GR IT LI LU NL SE**

(56) Documents cités:

**CHEMICAL ABSTRACTS, vol. 95, no. 4, 27 juillet 1981, page 400, résumé no. 31163w, Columbus, Ohio, US; O. POPOVYCH: "Solubility data. Guanidine tetraphenylborate-water system", & SOLUBILITY DATA SER. 1981, 18, 19**

(73) Titulaire: **SOLVAY & Cie (Société Anonyme)
Rue du Prince Albert, 33
B-1050 Bruxelles(BE)**

(72) Inventeur: **Callens, Roland
Flonkedreef 3
B-9810 Gent-Drongen(BE)**
Inventeur: **Collin, André
Rue du Pont Piraux 3A
B-6338 Ligny(BE)**

(74) Mandataire: **Lechien, Monique et al
Solvay Département de la Propriété Industrielle Rue de Ransbeek, 310
B-1120 Bruxelles(BE)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 297 641 B1

## Description

La présente invention concerne des composés guanidiniques comprenant un ion tétraphénylborate substitué, associé à un produit comprenant une fonction guanidinique, ainsi qu'un procédé d'obtention de tels composés qui peuvent être utilisés comme moyen pour solubiliser le produit dont la protection de la fonction guanidinique est assurée, notamment lors de la synthèse peptidique à partir d'acides aminés ou de peptides.

La demande de brevet allemand DOS 2716477 divulgue notamment des sels de guanidine N, N′,N″ substitués de formule générale :

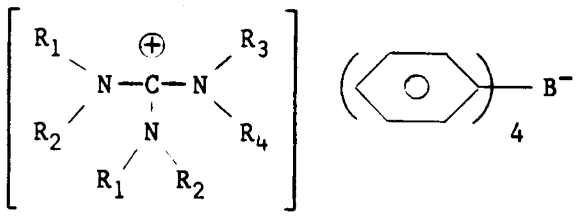

dans laquelle $R_1$, $R_2$, $R_3$ et $R_4$ représentent un radical aliphatique, cyclique, aliphatique arylé, aromatique ou hétérocyclique et dans laquelle, seul $R_1$ peut être un atome d'hydrogène.

Ces produits, protonés sur l'atome de carbone du groupement guanidinique substitué, sont synthétisés à partir d'un dérivé halogéné d'acide carbamique et de thiourée substituée et peuvent être utilisés comme catalyseurs, agents de protection des plantes et teintures pharmaceutiques.

Par ailleurs, des sels formés de l'anion tétrakis[3,5-bis (trifluorométhyl)phényl]borate et de certains cations diazonium tels que $ArN_2^+$ $ArNHR_2^+$ dans lesquels Ar représente un radical aryle et R un radical méthyle, sont également connus (Bulletin Chem. Soc. Jpn, 56, 796-801, 1983, vol. 56, N.3).

On a maintenant trouvé une nouvelle catégorie de composés dont la formule de structure est voisine de celle des produits précités mais dont toutes les valences des atomes d'azote de la fonction guanidinique à l'exception d'une seule sont saturées par des atomes d'hydrogène.

Plus particulièrement, les composés selon l'invention comportent une fonction guanidinique et un ion tétraphénylborate et répondent à la formule générale :

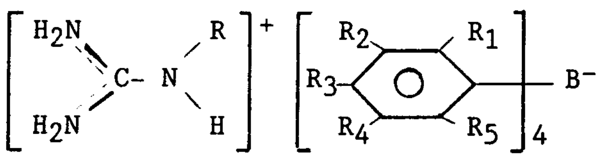

dans laquelle R représente un radical organique de formule générale :

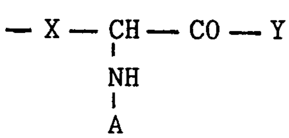

dans laquelle

- X représente un radical aliphatique linéaire, ramifié ou cyclique, substitué ou non, saturé ou non, contenant jusqu'à 25 atomes de carbone ;
- A représente un atome d'hydrogène; un radical aliphatique ou aromatique comportant des hétéroatomes ou non, tels que des groupements protecteurs ou des groupements d'activation; un ou plusieurs acides aminés liés par des liaisons peptidiques, dont certaines fonctions sont substituées ou non par des groupements protecteurs ou des groupements d'activation ;
- Y représente un groupement hydroxyle, un atome d'halogène, un radical aliphatique ou aromatique, comportant ou non des hétéroatomes, tels que des groupements protecteurs ou des groupements d'activation; un groupement aminé; un acide aminé ou un peptide dont certaines fonctions sont substituées ou non par des groupements protecteurs ou des groupements d'activation ainsi que par des groupements aminés de formule générale $NR_6R_7$ dans laquelle $R_6$ et $R_7$ représentent indépen-

damment l'un de l'autre un atome d'hydrogène ou un groupement alkyle comptant de 1 à 3 atomes de carbone ; et

- $R_1$, $R_2$, $R_3$, $R_4$ et $R_5$ représentent indépendamment les uns des autres, un groupement organique choisis parmi des radicaux alkyle, alkoxyalcényle ou alcényle comptant de 1 à 10 atomes de carbone et comportant ou non des hétéroatomes ou un atome d'hydrogène, l'un au moins des radicaux $R_1$, $R_2$, $R_3$, $R_4$ et $R_5$ étant différent d'un atome d'hydrogène.

De préférence :

- X représente un radical alkyle de formule générale $(CH_2)_n$ dans laquelle n est un nombre entier compris entre 1 et 10.
- A repésente un atome d'hydrogène, un acide aminé, un peptide ou un groupement protecteur.
- Y représente un groupement hydroxyle, un groupement protecteur, un groupement d'activation, un acide aminé ou un peptide.
- $R_1$, $R_2$, $R_3$, $R_4$ et $R_5$ représentent un atome d'hydrogène ou un radical alkyle comptant de 1 à 5 atomes de carbone et comportant éventuellement des hétéroatomes tels que des atomes d'halogène, notamment le chlore et le fluor.

De manière tout particulièrement préférée :

- X représente le radical $-(CH_2)_n-$ dans lequel n est compris entre 1 et 6.
- A représente un atome d'hydrogène, un groupement protecteur tel que notamment les groupements benzyloxycarbonyle (Z) ou tert-butyloxycarbonyle (t-Boc), un acide aminé ou un peptide éventuellement substitués par ces mêmes groupements protecteurs.
- Y représente un groupement hydroxyle, un groupement protecteur tel qu'un benzylester, un groupement d'activation tel que le N-hydroxysuccinimide, un acide aminé ou un peptide éventuellement substitués par des groupements protecteurs et/ou d'activation ou un groupement aminé.
- $R_1$, $R_2$, $R_3$, $R_4$ et $R_5$ représentent indépendamment les uns des autres un atome d'hydrogène ou un radical alkyle comptant de 1 à 3 atomes de carbone et des hétéroatomes tels que le chlore ou le fluor.

Enfin, de bons résultats ont été obtenus lorsque :

- X représente le radical $-(CH_2)_3-$.
- A représente un atome d'hydrogène, un acide aminé substitué ou non par un groupement protecteur ou un groupement protecteur tel que le benzyl oxycarbonyle ou le tert-butyloxycarbonyle, et
- Y un groupement hydroxyle ou un acide aminé substitué ou non par des groupements protecteurs et/ou d'activation,
- $R_1$, $R_3$ et $R_5$ représentent un atome d'hydrogène et $R_2$ et $R_4$ un groupement méthyle substitué tel que le groupement trifluoro méthyle.

Par acide aminé, on entend tout acide organique possédant au moins une fonction carboxylique et au moins une fonction amine primaire ou secondaire tel que les acides aminés naturels connus ou les acides aminés synthétiques. Par peptide, on entend tout peptide issu de toute association d'acides aminés naturels ou synthétiques.

Par groupement protecteur, on entend tout composé cité à cet effet dans la littérature et plus particulièrement par :

- M. BODANSZKY, Principles of Peptide Synthesis, 1984, Volume 16, Reactivity and Structure Concepts in Organic Chemistry,
- M. BODANSZKY, A. BODANSZKY, The Practice of Peptide Synthesis, 1984, Volume 21, Reactivity and Structure Concepts in Organic Chemistry.

A titre illustratif, les groupements protecteurs suivants peuvent être mis en oeuvre dans les composés de l'invention :

- des groupements protecteurs type acyle tels que notamment formyle, trifluoroacétyle, phthaloyle, 4-toluènesulfonyle, benzènesulfonyle, nitrophénylsulfényle, 2-nitrophénylsulfényle,
- des groupements protecteurs type uréthane aromatique tels que notamment le benzyloxycarbonyle substitué ou non tel que le p-chlorobenzyloxycarbonyle, le p-nitrobenzyloxycarbonyle, p-bromobenzyloxycarbonyle, p-méthoxybenzyloxycarbonyle, benzhydryloxycarbonyle, le 2-(4-biphénylyl)propyl(2)-oxycarbonyle, le 2-(3,5-diméthyloxyphényl)propyl(2)oxycarbonyle, le triphénylphosphonoéthyloxycarbonyle,
- des groupements protecteurs type uréthane aliphatique tels que notamment le tert-butyloxycarbonyle, le diisopropylméthoxycarbonyle, l'isopropyloxycarbonyle, l'éthoxycarbonyle, l'allyloxycarbonyle, le 9-fluorenylméthyloxycarbonyle, le 2-méthylsulfonyléthyloxycarbonyle, le 2,2,2-trichloroéthyloxycarbonyle,
- des groupements protecteurs type uréthane cycloalkyle tels que notamment le cyclopentyloxycarbonyle, l'adamantyloxycarbonyle, le cyclohexyloxycarbonyle, le tert-amyloxycarbonyle, l'isobornyloxycarbonyle,

- des groupements protecteurs type thiouréthane tels que notamment le phénylthiocarbonyle,
- des groupements protecteurs type alkyle tels que notamment le triphénylméthyle(trityle) et le benzyle,
- des groupements trialkylsilane tel que le triméthylsilane,
- des groupements alkoxy tel que notamment l'ester de méthyle, l'ester d'éthyle, le tert-butyl ester, le benzylester.

Par groupement d'activation, on entend tout groupement d'activation connu ou non, tel que ceux cités dans la littérature et plus particulièrement dans les articles de :

- M. BODANSZKY,
- M. BODANSZKY, A. BODANSZKY cités ci-avant.

Habituellement, on met en oeuvre comme groupement d'activation un groupement oxycarbonyle, oxycarboxyle, N-oxyimidoyle, imidazoyle tel que notamment le pivaloyle oxycarbonyle, le N-hydroxysuccinimidoyle, le dicyclohexylcarbodiimidoyle, le le 4-nitrophénylester.

Les composés préférés selon l'invention répondent aux formules :

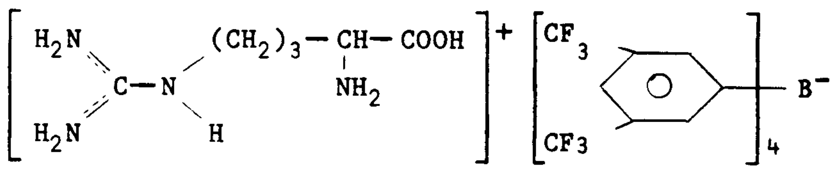

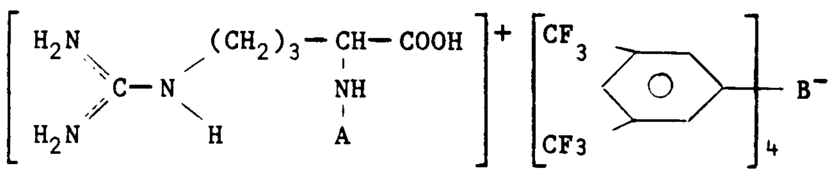

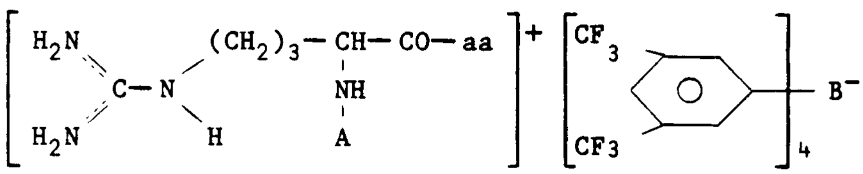

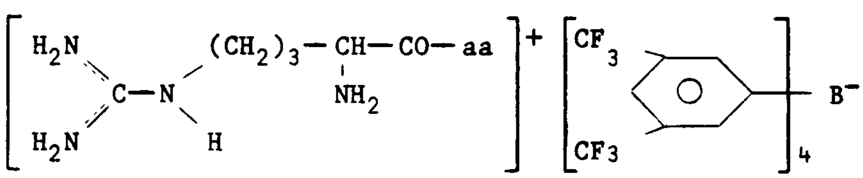

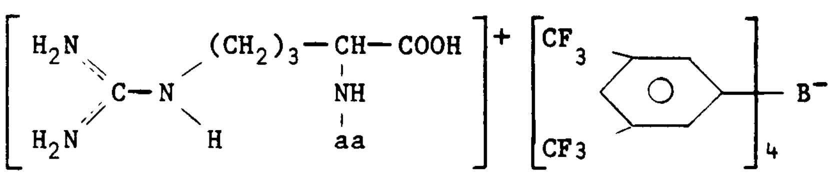

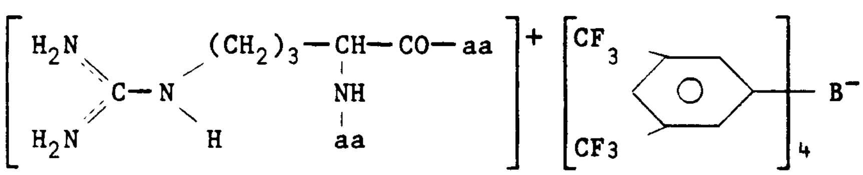

dans lesquelles aa représente un acide aminé ou un peptide lié par une liaison peptidique à l'arginine et dont les fonctions amines et carboxyliques sont éventuellement protégées ou substituées. Une telle protection est rendue nécessaire lorsque certains groupements fonctionnels, en particulier amine ou carboxylique, doivent être bloqués pour éviter qu'ils interviennent dans des réactions ultérieures lors de la mise en oeuvre du composé. La fonction carboxylique terminale de certains acides aminés ou peptides peut en outre être substituée par un groupement aminé tel que le groupement $-NH_2$ ou $-NH-CH_2-CH_3$.

De manière particulièrement préférée, aa représente un acide aminé.

Les composés selon l'invention peuvent être préparés par toute synthèse organique appropriée regroupant des réactions connues ou non et s'appliquant de façon générale ou de façon particulière à un seul composé déterminé ou à une famille de composés.

Un procédé ayant donné de bons résultats pour la préparation des composés selon l'invention consiste à mettre en oeuvre un sel de tétraphénylborate substitué et un produit comprenant un groupement guanidinique.

Les sels de tétraphénylborate substitué mis en oeuvre pour la synthèse des composés selon l'invention peuvent être formés à partir de n'importe quelle base inorganique ou organique.

Comme base organique, on utilise plus particulièrement une base organique azotée telle qu'une amine secondaire, tertiaire ou hétérocyclique. De bons résultats ont été obtenus avec la triéthylamine, la diisopropyléthylamine, la N-méthylmorpholine, la N-éthylmorpholine, la N-méthylpiperidine, la N-éthylpiperidine, la tri- n butylamine, la dicyclohexylamine et l'imidazole.

Comme base inorganique, on utilise habituellement un hydroxyde d'un métal alcalin ou alcalino-terreux, de préférence un hydroxyde d'un métal alcalin tel que notamment l'hydroxyde de sodium, l'hydroxyde de potassium et l'hydroxyde de magnésium.

De manière particulièrement préférée, on met en oeuvre un tétraphénylborate substitué de sodium, tel que le tétrakis [3,5-bis(trifluorométhyl)phényl]borate de sodium.

Le sel de tétraphénylborate substitué est mis en oeuvre dans la réaction en présence d'un solvant ou d'un mélange de solvants. Généralement, on met en oeuvre un seul solvant organique, polaire, tel que notamment le diméthylsulfoxide (DMSO), le N,N-diméthylformamide (DMF), la N-méthylpyrrolidone, l'acétonitrile ou un solvant chloré. De bons résultats ont été obtenus avec le N,N-diméthylformamide, le chloroforme, le dichlorométhane et le tétrachlorure de carbone.

La quantité de sel de tétraphénylborate substitué mis en oeuvre, peut varier dans de larges limites. En général, on met en oeuvre de 20 à 1 mole de sel de tétraphénylborate substitué par mole de produit comprenant la fonction guanidinique. De préférence, on met en oeuvre de 10 à 1 mole de sel de tétraphénylborate substitué. De manière particulièrement préférée, 1 mole de sel de tétraphénylborate substitué est mise en oeuvre par mole de produit comprenant la fonction guanidinique.

Les autres conditions opératoires utilisées dans le procédé pour la préparation des composés selon l'invention ne sont pas critiques pour l'invention. Ainsi, la pression à laquelle est effectué le procédé, est généralement comprise entre 0,1 et 10 bar et de bons résultats ont été obtenus à la pression atmosphérique. La température à laquelle est effectué le procédé, est habituellement comprise entre -60 et 100 ° C et peut varier selon la nature des réactifs et le composé que l'on veut finalement préparer.

Le procédé peut être réalisé dans tout appareillage conçu à cet effet.

Les composés de l'invention sont utilisables notamment comme intermédiaires de synthèse chimique.

Du fait de leur solubilité dans les solvants organiques, ils peuvent notamment être mis en oeuvre lors de leur propre couplage avec d'autres produits; en particulier lors de la synthèse de peptides à partir d'acides aminés telle que notamment décrit dans la demande de brevet européen 0184243 qui se rapporte à un procédé faisant intervenir un trialkylcyanosilane.

En outre, le fait que l'ion tétraphénylborate substitué est le contre-ion de la fonction guanidinique, dont il assure la protection, permet d'assurer la protection de cette fonction guanidinique lors de la réalisation de couplages sélectifs et d'obtenir ainsi des produits d'une plus grande pureté lors des synthèses. En effet, à la fin de la synthèse peptidique, l'ion tétraphénylborate substitué est facilement déplacé du produit comprenant la fonction guanidinique par toute méthode connue, soit par extraction sous forme d'un sel d'ammonium quaternaire, soit par précipitation dans l'eau sous forme d'un sel d'amine, ce qui permet de libérer d'une part la fonction guanidinique et d'autre part de récupérer l'ion tétraphénylborate substitué recyclable après un traitement adéquat.

Plus particulièrement, l'invention concerne l'utilisation de l'ion tétraphénylborate substitué comme moyen pour solubiliser dans des solvants organiques l'arginine et les peptides contenant des arginines non protégés dans la chaîne latérale.

Les composés selon l'invention, solubles dans les solvants organiques, présentent une excellente stabilité en milieu organique. Il est possible d'extraire l'arginine et les peptides contenant des arginines en milieu organique sous forme de tétraphénylborate substitué à partir de leur solution aqueuse. Cette propriété facilite les séparations des peptides par les techniques d'extraction en continu, telles que notamment la technique dite CCD ( Counter Current Distribution) qui met en oeuvre une extraction biphasique eau/milieu organique.

Les examples qui suivent, servent à illustrer l'invention sans toutefois en limiter la portée.

Dans ces exemples, les abréviations suivantes ont été utilisées :

- Arg : arginine
- Gly : glycine
- Leu : leucine
- Pro : proline
- O-Piv : pivaloyloxy(triméthyl acétyloxy)
- O-Succ : N-hydroxysuccinimide
- Z : groupement protecteur de type benzyloxycarbonyle
- Boc : groupement protecteur de type tert-butyloxycarbonyle
- t-Boc : groupement protecteur de type tert-butyloxycarbonyle
- DMF : N,N-diméthylformamide
- Et : groupement éthyle $-CH_2-CH_3$
- DCC : dicyclohexylcarbodiimide
- ONb : 4-nitrobenzylester
- HONb : N-hydroxy-5-norbornene-2,3-dicarboximide
- HBr : bromure d'hydrogène
- HOAc : acide acétique
- Mbh : bisparaméthoxybenzhydryle
- TFPB-Na : tétrakis[3,5-bis(trifluorométhyl)phényl]borate de sodium.

Exemple 1: Synthèse du composé formé du tripeptide t-Boc-Leu-Arg-Pro et de l'ion tétrakis [3,5-bis-(trifluorométhyl)phényl]borate

Dans un réacteur thermostatisé de 250 ml équipé d'un condenseur et d'un système d'agitation, on introduit successivement à température ambiante 112 g d'eau, 8,4 g (100 mmol) de $NaHCO_3$ et 8,66 g (20 mmol) de 2 HBr.Arg-Pro.

Après solubilisation complète du mélange et fin de dégagement de $CO_2$, on introduit rapidement 7,85 g (20 mmol) de t-Boc-Leu-ONb dissous dans 80 g de diméthoxyéthane.

Après 15 heures de mûrissage sous agitation à température ambiante, la solution réactionnelle est concentrée par évaporation à 40 °C sous pression réduite de manière à obtenir une solution résiduaire d'environ 100 ml. Ce résidu est amené à un pH de 2,5 par addition d'environ 100 ml de solution d'HCl 1N. On ajoute alors 200 ml de $CH_2Cl_2$ et 17,32 g (20 mmol) de TFPB-Na. La mélange obtenu est maintenu 30 min sous agitation vigoureuse à température ambiante puis laissé à décanter pendant 2 heures. La phase organique dense est soutirée puis soumise à évaporation à 40 °C sous pression réduite.

Le résidu sec de 28,7 g se présente sous forme d'un solide blanc pulvérulent et son contenu en t-Boc-Leu-Arg-Pro est de 29 % poids, ce qui représente une récupération de 17,2 mmol, soit un rendement de couplage de 86 % alors que le rendement n'est que de 60 % si on ne met pas en oeuvre l'ion tétrakis[3,5-bis(trifluorométhyl)phényl]borate. L'examen de la phase aqueuse montre que l'extraction du composé formé de l'ion tétrakis[3,5-bis(trifluorométhyl)phényl]borate et du tripeptide t-Boc-Leu-Arg-Pro a été quantitative.

Exemple 2 : Utilisation du composé obtenu à l'exemple 1

Dans un réacteur thermostatisé de 250 ml équipé d'un condenseur et d'un système d'agitation, 1mmol du composé obtenu à l'exemple 1 est dissous dans 10 ml d'HCl 0.1N puis on ajoute 10 ml de $CH_2Cl_2$.

Après avoir agité pendant 15 min, on soutire la phase organique, on la sèche sur $MgSO_4$ et l'on filtre. On récupère le filtrat et les lavages (5 ml) directement dans un réacteur de 50 ml que l'on munit d'un agitateur magnétique.

Après avoir ajouté 179 mg (1 mmol) de HONb, on refroidit le mélange à 0 °C et on ajoute 206 mg (1 mmol) de DCC. On laisse continuer l'activation pendant une heure à 0 °C et une heure à température ambiante puis on ajoute 301 mg (1 mmol) de Gly-Mbh.

On maintient sous agitation pendant 15 heures. A ce moment, aucun précipité n'est apparu. Pour épuiser le mélange réactionnel en HONb, on le lave 2 fois avec 20 ml de $NaHCO_3$ à 5 %. A l'aide d'une seringue, on transvase la phase dense et on l'évapore sous pression réduite à 30 °C.

En reprenant par de l'$Et_2O$ le dicyclohexylurée précipite. Après filtration, on concentre le filtrat et on le sèche sous vide poussé (1 mm Hg).

Le résidu pèse 1,5 g comprenant le tétrapeptide brut (t-Boc-Leu-Arg-Pro-Gly-NH-Mbh) d'une pureté peptidique d'environ 94 %, avec un rendement global de 86 %. L'identité du tétrapeptide est confirmée par NMR.

Exemple 3 : Synthèse du composé formé de l'ion tétrakis[3,5-bis(trifluorométhyl)phényl]borate et du peptide substitué Leu-Arg-Pro-Gly-$NH_2$

280 mg (0,5 mmol) de 2 HOAc.Leu-Arg-Pro-Gly-$NH_2$ sont dissous dans 5 ml d'eau. Puis la fonction α-aminée est libérée par addition de 5 ml de NaOH 0.1N (pH = 10,47). 443 mg de TFPB-Na sont mis en suspension dans 10 ml de $CH_2Cl_2$ et les deux solutions sont mélangées.

Après avoir agité pendant 15 min, la phase dense est soutirée et est concentrée à l'évaporateur rotatif.

Le séchage du résidu fournit quantitativement le composé formé de l'ion TFPB et du tétrapeptide substitué prêt à l'emploi.

La chaîne latérale de l'arginine reste protégée par l'ion TFPB tandis que la fonction α-aminée est libre, on peut donc coupler ce fragment, sans addition d'une base, à un acide aminé ou à un peptide.

**Revendications**

**Revendications pour les Etats contractants suivants : AT, BE, CH, DE, FR, GB, GR, IT, LI, LU, NL, SE**

**1.** Composés guanidiniques comprenant un ion tétraphénylborate caractérisés en ce qu'ils répondent à la formule générale :

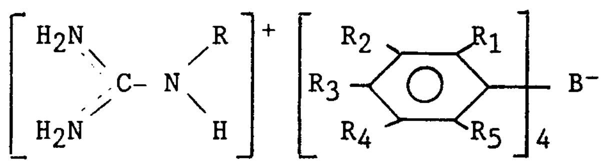

dans laquelle

- R représente un radical organique de formule générale :

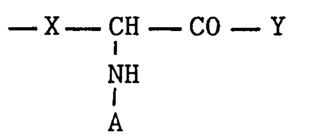

dans laquelle :

- X représente un radical aliphatique linéaire, ramifié ou cyclique, substitué ou non, saturé ou non, contenant jusqu'à 25 atomes de carbone ;
- A représente un atome d'hydrogène; un radical aliphatique ou aromatique comportant des hétéroatomes ou non, tels que des groupements protecteurs ou des groupements d'activation; un ou plusieurs acides aminés liés par des liaisons peptidiques, dont certaines fonctions sont substituées ou non par des groupements protecteurs ou des groupements d'activation ;
- Y représente un groupement hydroxyle, un atome d'halogène, un radical aliphatique ou aromatique, comportant ou non des hétéroatomes, tels que des groupements protecteurs ou des groupements d'activation; un groupement aminé; un acide aminé ou un peptide dont certaines fonctions sont substituées ou non par des groupements protecteurs ou des groupements d'activation ainsi que par des groupements aminés de formule générale $NR_6R_7$ dans laquelle $R_6$ et $R_7$ représentent indépendamment l'un de l'autre un atome d'hydrogène ou un groupement alkyle comptant de 1 à 3 atomes de carbone ; et
- $R_1$, $R_2$, $R_3$, $R_4$ et $R_5$ représentent indépendamment les uns des autres, un groupement organique choisis parmi des radicaux alkyle, alkoxyalcényle ou alcényle comptant de 1 à 10 atomes de carbone et comportant ou non des hétéroatomes ou un atome d'hydrogène, l'un au moins des radicaux $R_1$, $R_2$, $R_3$, $R_4$ et $R_5$ étant différent d'un atome d'hydrogène.

**2.** Composés selon la revendication 1 caractérisés en ce que :

- X représente le radical $-(CH_2)_3-$ ;
- A représente un atome d'hydrogène, un acide aminé ou un peptide substitué ou non par un groupement protecteur ou un groupement protecteur tel que le groupement benzyloxycarbonyle ou tert-butyloxycarbonyle;

- Y représente un groupement hydroxyle ou un acide aminé substitué ou non par des groupements protecteurs et/ou d'activation ;
- $R_1$, $R_3$ et $R_5$ représentent un atome d'hydrogène;
- $R_2$ et $R_4$ représentent un groupement méthyle substitué tel que le groupement trifluorométhyle.

**3.** Composés selon la revendication 2 caractérisés en ce qu'ils répondent aux formules

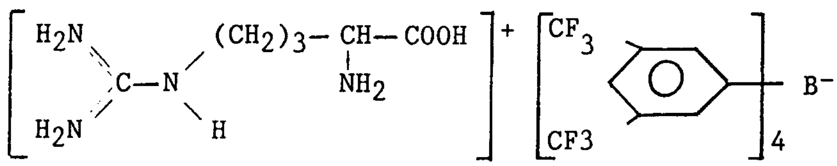

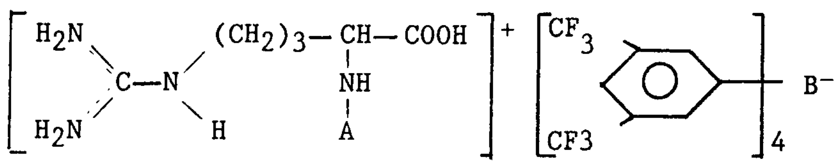

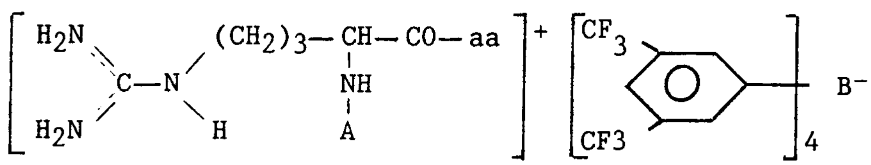

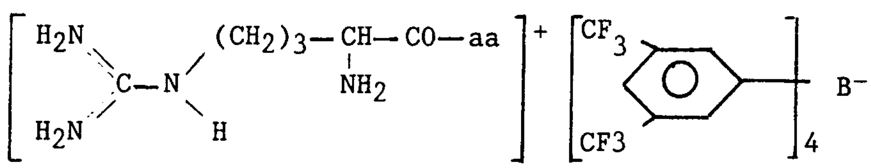

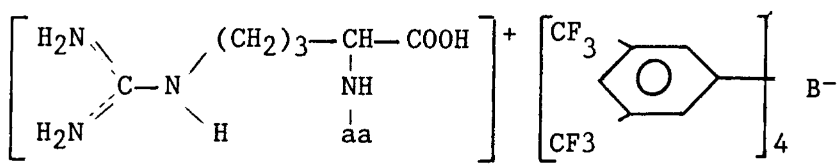

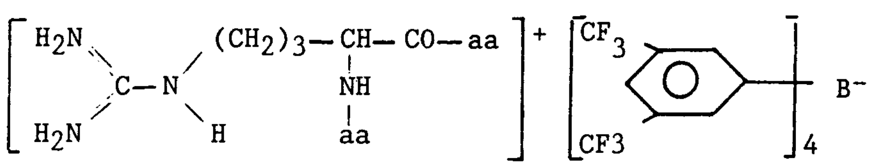

dans lesquelles aa représente un acide aminé ou un peptide lié par une liaison peptidique à l'arginine et dont les fonctions amines et carboxyliques sont protégées ou non ou substituées ou non ou dont la fonction carboxylique terminale est ou non en outre substituée par un groupement aminé tel que le groupement $-NH_2$ ou $-NH-CH_2-CH_3$.

**4.** Procédé pour la préparation des composés selon la revendication 1 caractérisé en ce qu'il met en oeuvre un sel de tétraphénylborate et un produit comprenant un groupement guanidique.

**5.** Procédé selon la revendication 4 caractérisé en ce que le sel de tétraphénylborate mis en oeuvre est formé à partir d'une base inorganique telle qu'un hydroxyde d'un métal alcalin ou alcalino-terreux.

**6.** Procédé selon la revendication 5 caractérisé en ce que la base inorganique est l'hydroxyde de sodium, l'hydroxyde de potassium ou l'hydroxyde de magnésium.

7. Procédé selon l'une quelconque des revendications 4 à 6 caractérisé en ce qu'on met en oeuvre un solvant tel que le N,N-diméthylformamide, le chloroforme, le dichlorométhane et le tétrachlorure de carbone.

8. Utilisation des composés selon l'une quelconque des revendications 1 à 3 lors de la synthèse peptidique par voie chimique.

**Revendications pour l'Etat contractant suivant : ES**

1. Procédé pour la préparation de composés guanidiniques comprenant un ion tétraphénylborate répondant à la formule générale :

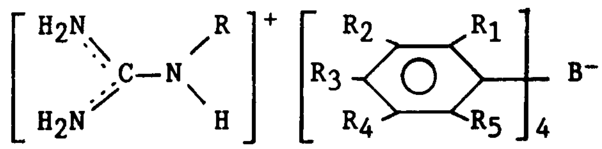

dans laquelle
- R représente un radical organique de formule générale :

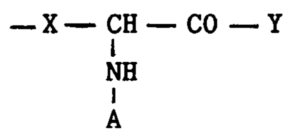

dans laquelle :
- X représente un radical aliphatique linéaire, ramifié ou cyclique, substitué ou non, saturé ou non, contenant jusqu'à 25 atomes de carbone ;
- A représente un atome d'hydrogène ; un radical aliphatique ou aromatique comportant des hétéroatomes ou on, tels que des groupements protecteurs ou des groupements d'activation ; un ou plusieurs acides aminés liés par des liaisons peptidiques, dont certaines fonctions sont substituées ou non par des groupements protecteurs ou des groupements d'activation ;
- Y représente un groupement hydroxyle, un atome d'halogène, un radical aliphatique ou aromatique, comportant ou non des hétéroatomes, tels que des groupements protecteurs ou des groupements d'activation ; un groupement aminé ; un acie aminé ou un peptide dont certaines fonctions sont substituées ou non par des groupements protecteurs ou des groupements d'activation ainsi que par des groupements aminés de formule générale $NR_6R_7$ dans laquelle $R_6$ et $R_7$ représentent indépendamment l'un de l'autre un atome d'hydrogène ou un groupement alkyle comptant de 1 à 3 atomes de carbone ; et
- $R_1$, $R_2$, $R_3$, $R_4$ et $R_5$ représentent indépendamment les uns des autres un groupement organique choisis parmi des radicaux alkyle, alkoxyalcényle ou alcényle comptant de 1 à 10 atomes de carbone et comportant ou non des hétéroatomes ou un atome d'hydrogène, l'un au moins des radicaux $R_1$, $R_2$, $R_3$, $R_4$ et $R_5$étant différent d'un atome d'hydrogène, caractérisé en ce qu'il met en oeuvre un sel de tétraphénylborate substitué et un produit comprenant un groupement guanidique.

2. Procédé selon la revendication 1 caractérisé en ce que :
- X représente le radical $-(CH_2)_3-$ ;
- A représente un atome d'hydrogène, un acide aminé substitué ou non par un groupement protecteur ou un groupement protecteur tel que le groupement benzyloxycarbonyle ou tert-butyloxycarbonyle ;
- Y représente un groupement hydroxyle ou un acide aminé substitué ou non par des groupements protecteurs et/ou d'activation ;
- $R_1$, $R_3$ et $R_5$ représentent un atome d'hydrogène;
- $R_2$ et $R_4$ représentent un groupement méthyle substitué tel que le groupement trifluorométhyle.

**3.** Procédé selon la revendication 1 ou 2 caractérisé en ce que l'on met en oeuvre un sel de tétraphénylborate formé à partir d'une base inorganique telle qu'un hydroxyde d'un métal alcalin ou alcalino-terreux.

**4.** Procédé selon la revendication 3 caractérisé en ce que l'on met en oeuvre corne base inorganique l'hydroxyde de sodium, l'hydroxyde de potassium ou l'hydroxyde de magnésium.

**5.** Procédé selon la revendication 1 ou 2 caractérisé en ce que l'on met en oeuvre le tétrakis [3,5-bis-(trifluorométhyl)phényl]borate de sodium.

**6.** Procédé selon l'une quelconque des revendications 1 à 5 caractérisé en ce qu'on met en oeuvre un solvant tel que le N,N-diméthylformamide, le chloroforme, le dichlorométhane et le tétrachlorure de carbone.

**7.** Utilisation des composés obtenus selon l'une quelconque des revendications 1 à 6 lors de la synthèse peptidique par voie chimique.

**Claims**

**Claims for the following Contracting States : AT, BE, CH, DE, FR, GB, GR, IT, LI, LU, NL, SE**

**1.** Guanidine-related compounds comprising a tetraphenylborate ion, characterised in that they correspond to the general formula:

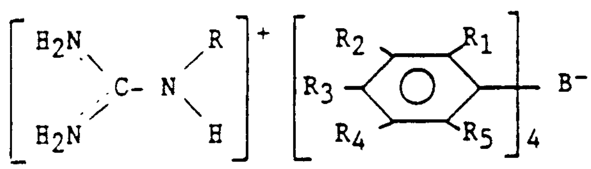

in which

- R denotes an organic radical of general formula:

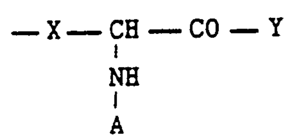

in which

- X denotes a linear, branched or cyclic, substituted or unsubstituted, saturated or unsaturated aliphatic radical, containing up to 25 carbon atoms,
- A denotes a hydrogen atom, an aliphatic or aromatic radical containing heteroatoms or otherwise, such as protective groups or activating groups, one or more amino acids bonded by peptide bonds, in which certain functional groups are substituted or unsubstituted by protective groups or activating groups;
- Y denotes a hydroxyl group, a halogen atom, an aliphatic or aromatic radical containing or not containing heteroatoms, such as protective groups or activating groups, an amino group, an amino acid or a peptide in which certain functional groups are substituted or unsubstituted by protective groups or activating groups and by amine groups of general formula $NR_6R_7$ in which $R_6$ and $R_7$ independently of each other denote a hydrogen atom or an alkyl group numbering from 1 to 3 carbon atoms; and
- $R_1$, $R_2$, $R_3$, $R_4$ and $R_5$ independently of each other denote an organic group chosen from alkyl, alkoxyalkenyl or alkenyl radicals numbering from 1 to 10 carbon atoms and containing or not containing heteroatoms or a hydrogen atom, at least one of the radicals $R_1$, $R_2$, $R_3$, $R_4$ and $R_5$ being other than a hydrogen atom.

**2.** Compounds according to Claim 1, characterised in that:

- X denotes the radical $-(CH_2)_3-$;
- A denotes a hydrogen atom, an amino acid or a peptide substituted or unsubstituted by a protective group or a protective group such as the benzyloxycarbonyl or tert-butoxycarbonyl group;
- Y denotes a hydroxyl group or an amino acid substituted or unsubstituted by protective and/or activating groups;
- $R_1$, $R_3$ and $R_5$ denote a hydrogen atom;
- $R_2$ and $R_4$ denote a substituted methyl group such as the trifluoromethyl group.

**3.** Compounds according to Claim 2, characterised in that they correspond to the formulae

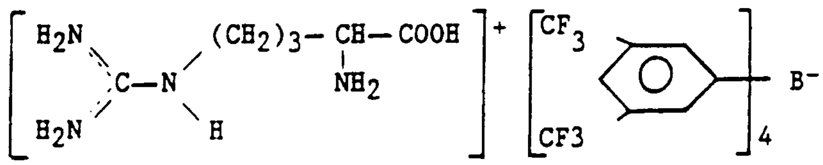

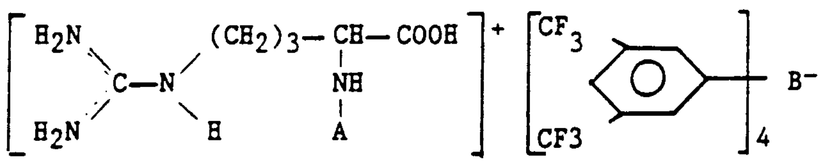

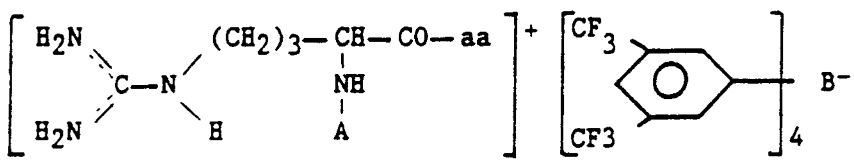

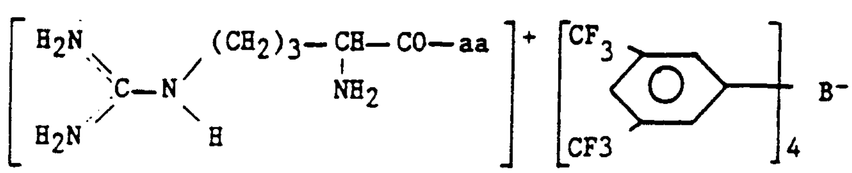

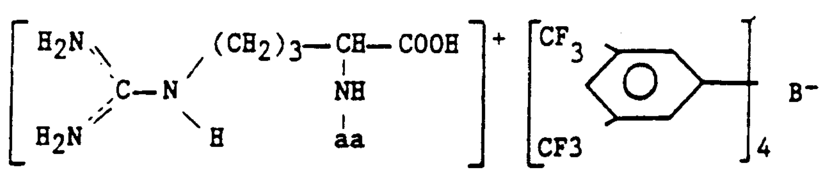

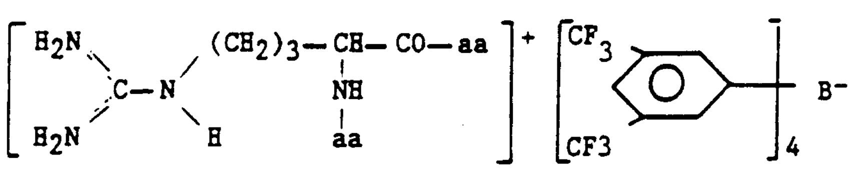

in which aa denotes an amino acid or a peptide bonded by a peptide bond to arginine and in which the amine and carboxylic functional groups are protected or otherwise or are substituted or otherwise or in which the terminal carboxylic functional group is additionally substituted or unsubstituted by an amino group such as the $-NH_2$ or $-NH-CH_2-CH_3$ group.

**4.** Process for the preparation of the compounds according to Claim 1, characterised in that a tetraphenylborate salt and a product containing a guanidic group are employed.

**5.** Process according to Claim 4, characterised in that the tetraphenylborate salt employed is formed from an inorganic base such as an alkali or alkaline-earth metal hydroxide.

**6.** Process according to Claim 5, characterised in that the inorganic base is sodium hydroxide, potassium hydroxide or magnesium hydroxide.

**7.** Process according to any one of Claims 4 to 6, characterised in that a solvent such as N,N-dimethylformamide, chloroform, dichloromethane and carbon tetrachloride is employed.

**8.** Use of the compounds according to any one of Claims 1 to 3 in peptide synthesis by a chemical route.

**Claims for the following Contracting State : ES**

**1.** Process for the preparation of guanidine-related compounds comprising a tetraphenylborate ion and corresponding to the general formula :

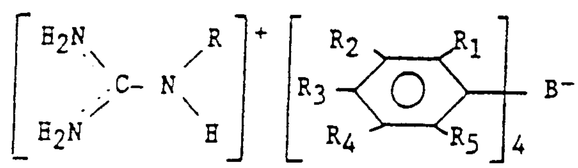

in which
- R denotes an organic radical of general formula :

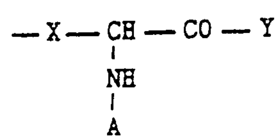

in which
- X denotes a linear, branched or cyclic, substituted or unsubstituted, saturated or unsaturated aliphatic radical, containing up to 25 carbon atoms,
- A denotes a hydrogen atom, an aliphatic or aromatic radical containing heteroatoms or otherwise, such as protective groups or activating groups, one or more amino acids bonded by peptide bonds, in which certain functional groups are substituted or unsubstituted by protective groups or activating groups;
- Y denotes a hydroxyl group, a halogen atom, an aliphatic or aromatic radical containing or not containing heteroatoms, such as protective groups or activating groups, an amino group, an amino acid or a peptide in which certain functional groups are substituted or unsubstituted by protective groups or activating groups and by amine groups of general formula $NR_6R_7$ in which $R_6$ and $R_7$ independently of each other denote a hydrogen atom or an alkyl group numbering from 1 to 3 carbon atoms; and
- $R_1$, $R_2$, $R_3$, $R_4$ and $R_5$ independently of each other denote an organic group chosen from alkyl, alkoxyalkenyl or alkenyl radicals numbering from 1 to 10 carbon atoms and containing or not containing heteroatoms or a hydrogen atom, at least one of the radicals $R_1$, $R_2$, $R_3$, $R_4$ and $R_5$ being other than a hydrogen atom, characterised in that a tetraphenylborate salt and a product containing a guanidic group are employed.

**2.** Process according to Claim 1, characterised in that :
- X denotes the radical $-(CH_2)-_3$;
- A denotes a hydrogen atom, an amino acid or a peptide substituted or unsubstituted by a protective group or a protective group such as the benzyloxycarbonyl or tert-butoxycarbonyl group;
- Y denotes a hydroxyl group or an amino acid substituted or unsubstituted by protective and/or activating groups;

- $R_1$, $R_3$ and $R_5$ denote a hydrogen atom;
- $R_2$ and $R_4$ denote a substituted methyl group such as the trifluoromethyl group.

**3.** Process according to Claim 1 or 2, characterised in that the tetraphenylborate salt employed is formed from an inorganic base such as an alkali or alkaline-earth metal hydroxide.

**4.** Process according to claim 3, characterised in that the inorganic base is sodium hydroxide, potassium hydroxide or magnesium hydroxide.

**5.** Process according to Claim 1 or 2, characterised in that sodium tetrakis [ 3,5-bis (trifluoromethyl)-phenyl] borate is employed.

**6.** Process according to any one of Claims 1 to 5, characterised in that a solvent such as N,N-dimethylformamide, chloroform, dichloromethane and carbon tetrachloride is employed.

**7.** Use of the compounds obtained according to any one of Claims 1 to 6 in peptide synthesis by a chemical route.

**Patentansprüche**
**Patentansprüche für folgende Vertragsstaaten : AT, BE, CH, DE, FR, GB, GR, IT, LI, LU, NL, SE**

**1.** Guanidin-Verbindungen, die ein Tetraphenylborat-Ion umfassen, dadurch gekennzeichnet, daß sie der allgemeinen Formel:

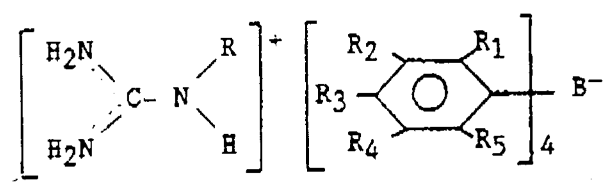

entsprechen, in der
- R einen organischen Rest der allgemeinen Formel:

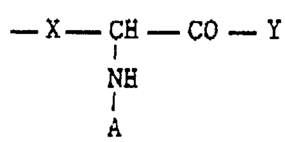

darstellt, in der
- X einen linearen, verzweigten oder ringförmigen, substituierten oder nicht-substituierten, gesättigten oder ungesättigten aliphatischen Rest darstellt, der bis zu 25 Kohlenstoffatome enthält;
- A ein Wasserstoffatom, einen aliphatischen oder aromatischen Rest, der Heteroatome, wie Schutzgruppierungen oder Aktivierungsgruppierungen, oder keine umfaßt, eine oder mehrere Aminosäuren, gebunden durch peptidbindungen, darstellt, wobei bestimmte Funktionen durch Schutzgruppierungen oder Aktivierungsgruppierung substituiert sind oder nicht;
- Y eine Hydroxylgruppierung, ein Halogenatom, einen aliphatischen oder aromatischen Rest, der Heteroatome, wie Schutzgruppierungen oder Aktivierungsgruppierungen, oder keine, umfaßt, eine Aminogruppierung, eine Aminosäure oder ein Peptid darstellt, wobei bestimmte Funktionen substituiert sind oder nicht durch Schutzgruppierungen oder Aktivierungsgruppierungen, ebenso wie durch Aminogruppierungen der allgemeinen Formel $NR_6R_7$, in der $R_6$ und $R_7$ unabhängig voneinander ein Wasserstoffatom oder eine Alkylgruppierung mit 1 bis 3 Kohlenstoffatomen darstellen; und
- $R_1$, $R_2$, $R_3$, $R_4$ und $R_5$ unabhängig voneinander eine organische Gruppierung, die unter den Alkyl-, Alkoxyalkenyl- oder Alkenyl-Resten mit 1 bis 10 Kohlenstoffatomen ausgewählt ist und Heteroatome oder keine aufweist, oder ein Wasserstoffatom darstellen, wobei wenigstens einer der Reste $R_1$, $R_2$, $R_3$, $R_4$ und $R_5$ verschieden von einem Wasserstoffatom ist.

**2.** Verbindungen nach Anspruch 1, dadurch gekennzeichnet, daß:
- X den Rest -$(CH_2)_{-3}$ darstellt;
- A ein Wasserstoffatom, eine Aminosäure oder ein Peptid, substituiert oder nicht durch eine Schutzgruppierung oder eine schutzgruppierung, wie die Benzyloxycarbonyl- oder tert-Butyloxycarbonyl-Gruppierurg, darstellt;
- Y eine Hydroxylgruppierung oder eine Aminosäure substituiert oder nicht durch Schutz- und/oder Aktivierungsgruppierungen darstellt;
- $R_1$, $R_3$ und $R_5$ ein Wasserstoffatom darstellen;
- $R_2$ und $R_4$ eine substituierte Methylgruppierung, wie die Trifluormethylgruppierung darstellen.

**3.** Verbindungen nach Anspruch 2, dadurch gekennzeichnet, daß sie den folgenden Formeln entsprechen:

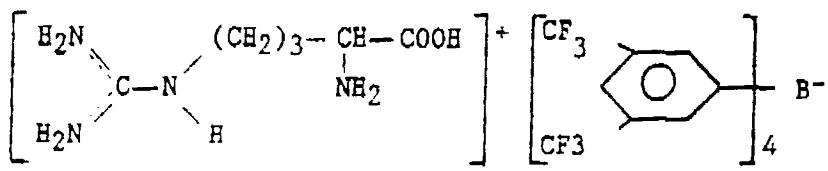

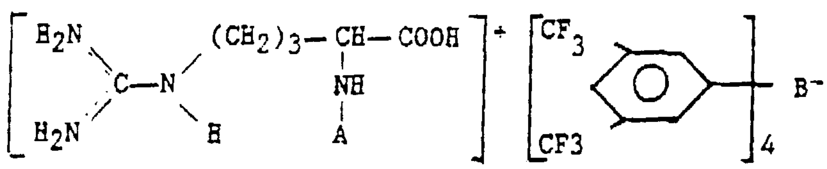

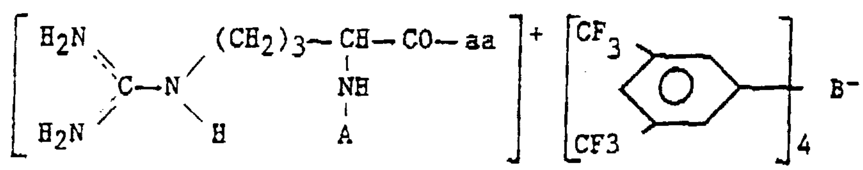

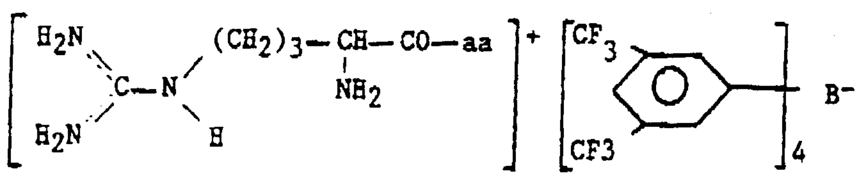

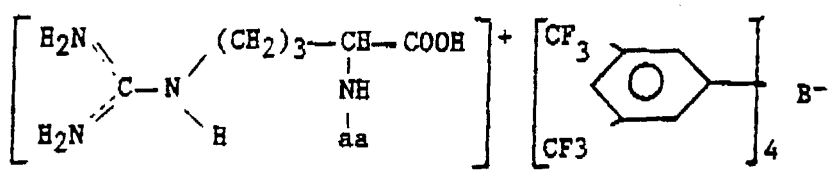

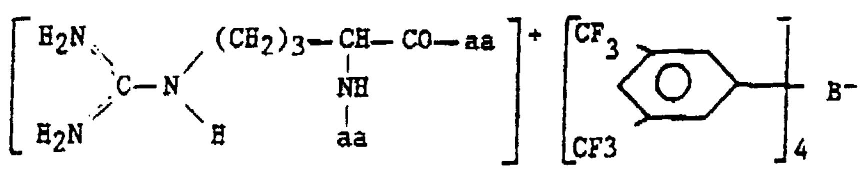

in denen aa eine Aminosäure oder ein Peptid darstellt, gebunden durch eine Peptidbindung an das Arginin und dessen Amino- und Carboxyl-Funktionen geschützt sind oder nicht oder substituiert sind oder nicht oder dessen End-Carboxyl-Funktion außerdem durch eine Aminogruppierung, wie die Gruppierung -$NH_2$ oder -$NH$-$CH_2$- $CH_3$ substituiert ist oder nicht.

**4.** Verfahren zur Herstellung von Verbindungen nach Anspruch 1, dadurch gekennzeichnet, daß ein Tetraphenylboratsalz und ein Produkt, umfassend eine Guanidin-Gruppierung, eingesetzt wird.

**5.** Verfahren nach Anspruch 4, dadurch gekennzeichnet, daß das eingesetzte Tetraphenylboratsalz ausgehend von einer anorganischen Base, wie einem Alkalimetall- oder Erdalkalimetall-Hydroxyd, gebildet wird.

**6.** Verfahren nach Anspruch 5, dadurch gekennzeichnet, daß die anorganische Base Natriumhydroxyd, Kaliumhydroxyd oder Magnesiumhydroxyd ist.

**7.** Verfahren nach einem der Ansprüche 4 bis 6, dadurch gekennzeichnet, daß man ein Lösungsmittel, wie N,N-Dimethylformamid, Chloroform, Dichlormethan und Tetrachlorkohlenstoff einsetzt.

**8.** Verwendung der Verbindungen nach einem der Ansprüche 1 bis 3 bei der Peptid-Synthese auf chemischem Wege.

**Patentansprüche für folgenden Vertragsstaaten : ES**

**1.** Verfahren zur Herstellung von Guanidin-Verbindungen, die ein Tetraphenylborat-Ion umfassen, die der allgemeinen Formel :

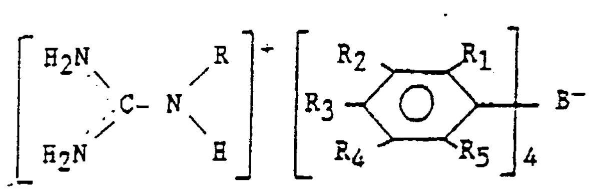

entsprechen, in der

- R einen organischen Rest der allgemeinen Formel :

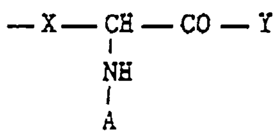

darstellt, in der

- x einen linearen, verzweigten oder ringförmigen, substituierten oder nicht-substituierten, gesättigten oder ungesättigten aliphatischen Rest darstellt, der bis zu 25 Kohlenstoffatome enthält;
- A ein Wasserstoffatom, einen aliphatischen oder aromatischen Rest, der Heteroatome, wie Schutzgruppierungen oder Aktivierungsgruppierungen, oder keine umfaßt, eine oder mehrere Aminosäuren, gebunden durch Peptidbindungen, darstellt, wobei bestimmte Funktionen durch Schutzgruppierungen oder Aktivierungsgruppierung substituiert sind oder nicht;
- Y eine Hydroxylgruppierung, ein Halogenatom, einen aliphatischen oder aromatischen Rest, der Heteroatome, wie Schutzgruppierungen oder Aktivierungsgruppierungen, oder keine, umfaßt, eine Aminogruppierung, eine Aminosäure oder ein Peptid darstellt, wobei bestimmte Funktionen substituiert sind oder nicht durch Schutzgruppierungen oder Aktivierungsgruppierungen, ebenso wie durch Aminogruppierungen der allgemeinen Formel $NR_6R_7$, in der $R_6$ und $R_7$ unabhängig voneinander ein wasserstoffatom oder eine Alkylgruppierung mit 1 bis 3 Kohlenstoffatomen darstellen; und
- $R_1$, $R_2$, $R_3$, $R_4$ und $R_5$ unabhängig voneinander eine organische Gruppierung, die unter den Alkyl-, Alkoxyalkenyl- oder Alkenyl-Resten mit 1 bis 10 Kohlenstoffatomen ausgewählt ist und Heteroatome oder keine aufweist, oder ein Wasserstoffatom darstellen, wobei wenigstens einer der Reste $R_1$, $R_2$, $R_3$, $R_4$ und $R_5$ verschieden von einem Wasserstoffatom ist, dadurch gekennzeichnet, daß ein Tetraphenylboratsalz und ein Produkt, umfassend eine Guanidin-Gruppierung, einsgesetzt wird.

**2.** Verfahren nach Anspruch 1, dadurch gekennzeichnet, daß:

- X den Rest $-(CH_2)-_3$ darstellt;
- A ein Wasserstoffatom, eine Aminosäure oder ein Peptid, substituiert oder nicht durch eine

Schutzgruppierung oder eine Schutzgruppierung, wie die Benzyloxycarbonyl- oder tert-Butyloxycarbonyl-Gruppierung, darstellt;

- Y eine Hydroxylgruppierung oder eine Aminosäure substituiert oder nicht durch Schutz- und/oder Aktivierungsgruppierungen darstellt;
- $R_1$, $R_3$ und $R_5$ ein Wasserstoffatom darstellen;
- $R_2$ und $R_4$ eine substituierte Methylgruppierung, wie die Trifluormethylgruppierung darstellen.

**3.** Verfahren nach Anspruch 1 oder 2, dadurch gekennzeichnet, daß das eingesetzte Tetraphenylboratsalz ausgehend von einer anorganischen Base, wie einem Alkalimetall- oder Erdalkalimetall-Hydroxyd, gebildet wird.

**4.** Verfahren nach Anspruch 3, dadurch gekennzeichnet, daß die anorganische Base Natriumhydroxyd, Kaliumhydroxyd oder Magnesiumhydroxyd ist.

**5.** Verfahren nach anspruch 1 oder 2, dadurch gekennzeichnet, daß Natrium Tetrakis [3,5 bis (trifluoromethyl)phenyl] borat eingesetzt wird.

**6.** Verfahren nach einem der Ansprüche 1 bis 5, dadurch gekennzeichnet, daß man ein Lösungsmittel, wie N,N-Dimethylformamid, Chloroform, Dichlormethan und Tetrachlorkohlenstoff einsetzt.

**7.** Verwendung der Verbindungen nach einem der Ansprüche 1 bis 6 herstellte bei der Peptid-Synthese auf chemischem Wege.